# EUROPÄISCHE PATENTANMELDUNG

(11) **EP 4 379 186 A1**
(43) Veröffentlichungstag der Anmeldung: **05.06.2024**
(21) Anmeldenummer: 22210240.2
(22) Anmeldetag: 29.11.2022
(51) Int. Cl.: E21B 49/08, G01N 33/18, G01N 33/28, G01N 1/22, G01N 33/00

(54) **SONDE ZUR GASANALYSE IN BOHRLÖCHERN, GASANALYSESYSTEM UND VERFAHREN ZUR GASANALYSE IN EINEM BOHRLOCH**

(71) Anmelder: Solexperts AG, 8617 Mönchaltorf (CH)
(72) Erfinder: Helmlinger, Benjamin, 54000 Nancy (FR); Laurent, Alain, 86600 Celle l' Evescault (FR); de Donato, Philippe, 54600 Villers-lès-Nancy (FR); Pironon, Jacques, 54500 Vandoeuvre-lès-Nancy (FR); Lettry, Yannick, 2340 Le Noirmont (CH)
(74) Vertreter: Zimmermann & Partner Patentanwälte mbB

(57) **Zusammenfassung**

Sonde (100) zur Gasanalyse in Bohrlöchern (200) mit einem in das Bohrloch (200) einführbaren Sondenkörper (110) mit zumindest einer Öffnung (112) zum Einlassen von Fluid (210) aus einem Außenraum des Sondenkörpers (110) in einen Innenraum (114) des Sondenkörpers (110), einem in dem Innenraum (114) des Sondenkörpers (110) vorgesehenen Gaspermeator (120) mit einem Sintermetallträger (122) mit einem innenliegenden Gaskanal (126) und einer außenseitig auf dem Sintermetallträger (122) angeordneten, semipermeable Membran (124). Der Gaspermeator (120) ist zur Diffusion von Gasen von dem Fluid (210) in den innenliegenden Gaskanal (126) eingerichtet.

## Beschreibung

Die Erfindung liegt auf dem Gebiet der Bohrung, insbesondere der Tiefbohrung, sowie der Analyse von Stoffen, insbesondere Gasen, in einem Bohrloch.

Geologische Bohrungen mit einer Tiefe von über 500 Metern sind als Tiefbohrungen bekannt. Tiefbohrungen werden häufig als Vertikalbohrungen durchgeführt, jedoch können auch Horizontalbohrungen zu Bohrlöchern führen, in denen die Bedingungen innerhalb des Bohrlochs zumindest abschnittsweise denen von Tiefbohrungen ähneln. Bohrungen können für verschiedene Zwecke durchgeführt werden, beispielsweise zur Untersuchung der geologischen Verhältnisse, zur Erkundung und/oder Förderung potentieller Lagerstätten von Erdöl, Erdgas, natürlichem Wasserstoff, Edelgasen wie Helium, Erz, Salz, Grundwasser, Erdwärme und/oder mineralischen Rohstoffen, oder zur Errichtung von Untergrundspeichern, sowie Wirtsgesteinen von potentiellen Standorten von CO₂ Sequestrierungsprojekten oder geologischen Tiefenlager für radioaktive Abfälle.

Oftmals ist es von Interesse, die chemische Zusammensetzung des Grundwassers in verschiedenen Abschnitten z.B. in den wasserführenden Horizonten im Bohrloch zu bestimmen. Bei der Bestimmung der chemischen Zusammensetzung der Grundwässer kann es vorteilhaft sein, die im Grundwasser gelösten Gase zu bestimmen. Bei den gelösten Gasen kann es sich beispielsweise um Methan, CO₂, Wasserstoff, Stickstoff, Edelgase wie Argon, Neon Krypton oder Helium, Schwefelwasserstoff und/oder weitere Gase wie etwa Kohlenwasserstoffe handeln.

Üblicherweise umfasst die Analyse des Grundwassers eine Probenahme aus dem Bohrloch und die anschließende Analyse des Grundwassers an der Oberfläche. Probenahmen sind zeit- und arbeitsintensiv und sind zudem in Bezug auf die Konzentrationsmessung gelöster Gase fehleranfällig, beispielsweise kommt es aufgrund des Druckabfalls zwischen Probenahme und Analyse oft zu Ausgasungen, wodurch das Analyseergebnis beeinträchtigt werden kann.

Es besteht somit Bedarf an einer Sonde und Analysemethode zur Bestimmung der gelösten Gase in einem Bohrloch. Die hier vorgeschlagenen Apparate und Methoden lösen das genannte Problem zumindest teilweise.

Aufgabe der Erfindung ist es, eine Sonde in ein Bohrloch einzuführen, sodass eine Analyse der gelösten Gase in einem die Sonde umgebenden Fluid *in situ* ermöglicht wird.

Die Aufgabe wird mit einer Sonde nach Anspruch 1 und einem Verfahren nach dem nebengeordneten Anspruch gelöst. Vorteilhafte Weiterbildungen ergeben sich aus den Unteransprüchen und der folgenden Beschreibung.

Gemäß einem Aspekt wird eine Sonde zur Gasanalyse in Bohrlöchern beschrieben. Die Sonde umfasst einen in das Bohrloch einführbaren Sondenkörper mit zumindest einer Öffnung zum Einlassen von Fluid aus einem Außenraum des Sondenkörpers in einen Innenraum des Sondenkörpers, sowie einen in dem Innenraum des Sondenkörpers vorgesehenen Gaspermeator. Der Gaspermeator umfasst einen Sintermetallträger mit einem innenliegenden Gaskanal und eine außenseitig auf dem Sintermetallträger angeordnete, semipermeable Membran. Der Gaspermeator ist zur Diffusion von Gasen von dem Fluid in den innenliegenden Gaskanal eingerichtet.

Gemäß einem Aspekt wird ein Verfahren zur Gasanalyse in einem Bohrloch mit einer Sonde nach einer der hierin beschriebenen Ausführungsformen beschrieben. Das Verfahren umfasst das Einführen der Sonde in das Bohrloch an eine vorgegebene Position, das Abwarten einer Diffusion von Gasen von einem in dem Bohrloch an der vorgegebenen Position vorhandenen Fluid über die semipermeable Membran in den Gaskanal der Sonde, und die Analyse des in dem Gaskanal vorhandenen Gasgemisches.

Gemäß einem Aspekt ist ein Bohrloch beschrieben. Bei dem Bohrloch kann es sich um ein Bohrloch handeln, das mit einer (Sub)Horizontal- und/oder Vertikalbohrung erzeugt wurde. Das Bohrloch kann ein mittels Tiefbohrung hergestelltes Bohrloch sein. Insbesondere können in dem Bohrloch, beispielsweise an einer Stelle in dem Bohrloch, Bedingungen herrschen, durch die die Verwendung von bekannten Gas- und/oder Fluid-Analysegeräten erschwert oder sogar unmöglich ist. Beispielsweise können in dem Bohrloch Drücke von mehr als 50 bar, mehr als 60 bar, mehr als 70 bar, mehr als 80 bar, mehr als 90 bar, mehr als 100 bar, mehr als 110 bar, oder sogar etwa 120 bar herrschen. Die genannten Drücke können beispielsweise hydrostatische Drücke sein und durch die Tiefe des Bohrloches zustande kommen. Beispielsweise kann das Bohrloch eine Tiefe von mehr als 500 m, mehr als 600 m, mehr als 700 m, mehr als 800 m, mehr als 900 m, oder sogar mehr als 1000 m haben. Beispielsweise können in dem Bohrloch Temperaturen von mehr als 50°C, mehr als 60°C, mehr als 70°C, oder sogar mehr als 80°C herrschen.

Gemäß einem Aspekt kann bei einem mittleren geothermischen Gradienten (30°C/km) die Temperatur im Bohrloch bei einer Tiefe von ca. 1000m etwa 40°C erreichen. Bei einem hohen geothermischen Gradienten können jedoch auch Temperaturen von 80°C erreicht werden. Diese Bedingungen können für *in-situ* Messungen mit bekannten Systemen nachteilig sein.

Gemäß einem Aspekt kann das Bohrloch ein Bohrloch mit einem kleinen Durchmesser sein. Insbesondere kann das Bohrloch einen Durchmesser von 76 mm (NQ) aufweisen. Der Durchmesser des Bohrlochs kann durch die Verwendung eines Bohrkopfes mit 76 mm bei der Bohrung des Bohrloches definiert sein. Gleichermaßen kann das Bohrloch auch einen größeren Durchmesser aufweisen, beispielsweise HQ (96 mm) oder PQ (122 mm), oder 8.5" (216 mm).

Gemäß einem Aspekt ist die hierin beschriebene Sonde vorteilhafterweise dafür geeignet, in ein Bohrloch und/oder eine Stelle in einem Bohrloch mit den genannten Eigenschaften eingeführt zu werden und die hierin beschriebene Gasanalyse auszuführen.

Gemäß einem Aspekt ist ein Sintermetallträger beschrieben. Der Sintermetallträger kann als Hohlkörper gestaltet sein. Der Sintermetallträger kann einen Gaskanal beinhalten. Der Gaskanal kann sich entlang einer Achse zwischen einem offenen ersten Ende und einem offenen zweiten Ende des Sintermetallträgers erstrecken. Mehrere Gaskanäle mehrerer Sintermetallträger können zu einem gemeinsamen Gaskanal eines Gaspermeators verbunden sein. Der Gaspermeator kann zumindest einen, sowie eine Vielzahl von Sintermetallträgern umfassen.

Der Sintermetallträger kann aus einem gesinterten Edelstahl gefertigt sein. Insbesondere kann der Sintermetallträger porös sein. Der Sintermetallträger kann dazu geeignet sein, einen Gasfluss durch einen gesinterten Teil des Sintermetallträgers zu ermöglichen. Als Gasfluss kann ein indirekter Gasfluss, beispielsweise eine Diffusion verstanden werden. Als Gasfluss kann ein Gasfluss durch Poren des Sintermetallträgers verstanden werden. Beispielsweise kann ein Gas von einer Außenseite des Sintermetallträgers durch den Sintermetallträger in einen Innenraum des Sintermetallträgers möglich sein. Insbesondere kann ein Gas von einer Außenseite des Sintermetallträgers durch Poren des Sintermetallträgers in einen innenliegenden Gaskanal des Sintermetallträgers wandern, strömen und/oder, diffundieren. Der Sintermetallträger kann ein Sintermetall-Filter sein.

In Ausführungsformen kann der Sintermetallträger eine Porengröße von 0,1 µm bis 100 µm aufweisen, beispielsweise 0,2 µm bis 50 µm, beispielsweise 0,5 µm bis 10 µm, wie etwa 1 µm, 2 µm, 5 µm, oder 10 µm. Die Porengröße kann im Wesentlichen einheitlich sein. Die Porengröße kann im Wesentlichen in einem Porengrößenbereich, wie beispielsweise einem der genannten Porengrößenbereiche, liegen. Die Porengröße kann eine in den genannten Porengrößenbereichen liegende, beispielsweise durchschnittliche, Porengröße sein. Die Porengröße kann durch die Verwendung von Partikeln mit einer definierten, beispielsweise einheitlichen, Form und Größe als Sintermaterial definiert sein.

In Ausführungsformen kann der Sintermetallträger aus einem gesinterten Edelstahl gefertigt sein. Beispielsweise kann der Sintermetallträger aus gesinterten Edelstahl-Partikel gefertigt sein. Die Edelstahl-Partikel können eine oder mehrere Edelstahl-Legierung wie beispielsweise AISL 304 L, AISL 316 L; AISL 904 L, AISL 310 L, Hastelloy C 22, Hastelloy C 276, Inconel 600, Iconel 625 umfassen und/oder aus der Legierung oder Kombinationen daraus gefertigt sein. Vorteilhafterweise kann die Edelstahl-Legierung inert gegenüber Gasen wie beispielsweise Methan, CO₂, Wasserstoff, Schwefelwasserstoff und/oder weitere Gase wie etwa Kohlenwasserstoffe sein, insbesondere bei den in einem Bohrloch zu erwartenden Bedingungen, insbesondere bei einer Temperatur von mindestens 70 °C oder mehr und/oder einem Druck von 90 bar oder mehr.

Gemäß einem Aspekt ist eine semipermeable Membran beschrieben. Die semipermeable Membran kann für Gase durchlässig sein, insbesondere für Gase wie Methan, CO₂, Wasserstoff, Stickstoff, Edelgase wie Argon, Neon, Krypton oder Helium, Schwefelwasserstoff und/oder weitere Gase wie etwa Kohlenwasserstoffe. Beispielsweise kann die semipermeable Membran eine Diffusion der Gase durch die Membran ermöglichen. Die semipermeable Membran kann für Flüssigkeiten, insbesondere Wasser und/oder wässrige Lösungen, undurchlässig sein. Die semipermeable Membran kann für in der Flüssigkeit gelöste Gase durchlässig sein, insbesondere können in der Flüssigkeit gelöste Gase durch die Membran diffundieren und nach der Diffusion gasförmig vorliegen.

In Ausführungsformen kann die semipermeable Membran aus einem Polymer gefertigt sein, insbesondere einem Silikon-basierten Polymer, wie beispielsweise einem Poly(organo)siloxan, Polymethylsiloxan, Silikonkautschuk, Silikonelastomer, und/oder Flourosilikon. Beispielsweise kann die semipermeable Membran einen Silikonschlauch umfassen. Ein geeigneter Silikonschlauch kann beispielsweise ein Schlauch der Produktfamilie Tygon^{®} SPT-3350 (Saint-Gobain Performance Plastics) in der am Anmeldetag dieser Schrift verfügbaren Form sein. Vorteilhafterweise können Silikon-basierte Polymere dazu geeignet sein, eine Gasdiffusion insbesondere bei den in einem Bohrloch zu erwartenden Bedingungen, insbesondere bei einer Temperatur von mindestens 70 °C oder mehr und/oder einem Druck von 90 bar oder mehr zu ermöglichen.

Gemäß einem Aspekt ist ein Trägergas beschrieben. Das Trägergas kann ein beliebiges inertes Gas sein, das sich von einem der zu analysierenden Gase unterscheidet. Beispielsweise kann das Trägergas, Stickstoff oder ein Edelgas wie beispielsweise Argon sein.

Nachfolgend werden einige Ausführungsbeispiele der Erfindung anhand der beiliegenden Zeichnungen erläutert. Die Zeichnungen zeigen:
- Figur 1: Eine schematische Zeichnung einer Sonde gemäß einer Ausführungsform;
- Figur 2: Schematische Schnittzeichnungen der in Fig. 1 gezeigten Sonde in einem Bohrloch;
- Figur 3: Eine schematische Schnittzeichnung einer Endplatte einer Sonde gemäß einer Ausführungsform;
- Figur 4: Eine schematische Schnittzeichnung eines Gasanalysesystems gemäß einer Ausführungsform; und
- Figur 5: Ein Verfahren zur Gasanalyse in einem Bohrloch gemäß einer Ausführungsform.

Im Folgenden wir die Erfindung anhand von Ausführungsformen und Weiterbildungen beschrieben. Aspekte, Ausführungsformen und Weiterbildungen können miteinander kombiniert werden, um zu weiteren Ausführungsformen zu gelangen. In den Figuren werden dieselben Bezugszeichen, sofern angemessen, für gleiche oder gleichartige Merkmale und/oder Baugruppen verwendet. Zur besseren Erkennbarkeit sind für Baugruppen, die aus gleichen oder ähnlichen Komponenten bestehen, in den Zeichnungen teilweise nur manche der Komponenten mit Bezugszeichen versehen. In der folgenden Beschreibung sind spezifische Ausführungsformen beispielhaft beschreiben.

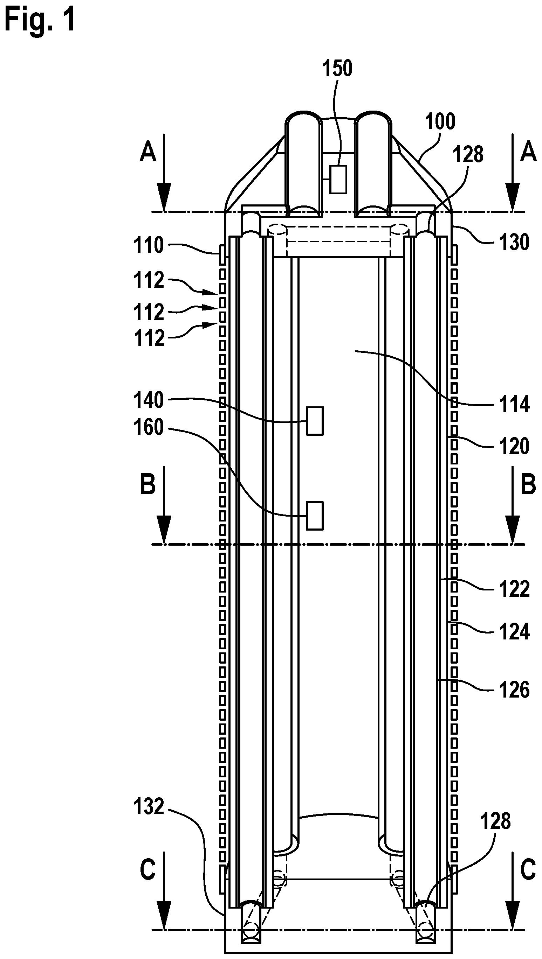

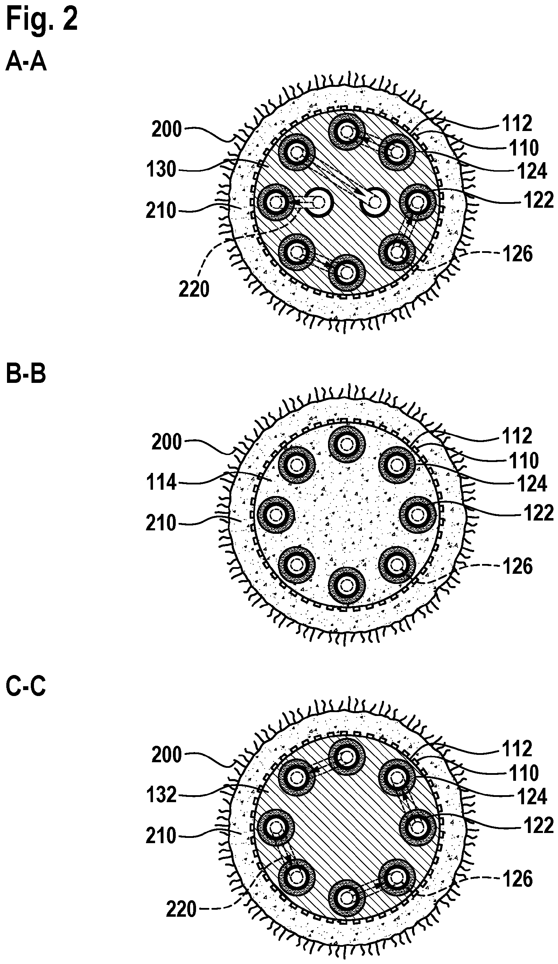

Fig. 1 zeigt eine Sonde 100 in einer Längsschnittzeichnung. Fig. 2 zeigt Querschnittzeichnungen der Sonde 100 entlang der in Fig. 1 gezeigten Ebenen A-A, B-B, C-C. Die Sonde 100 umfasst einen Sondenkörper 110. Der Sondenkörper 110 kann im Wesentlichen die Außenfläche der Sonde definieren. Der Sondenkörper 110 kann eine im Wesentlichen zylindrische Form aufweisen. Sofern hierin ein Durchmesser der Sonde 100 beschrieben wird, ist darunter, sofern nicht anders erwähnt, der Durchmesser der im Wesentlichen zylindrischen Form zu verstehen. Vorteilhafterweise können die Grundflächen des Sondenkörpers zumindest teilweise abgerundet oder angeschrägt sein, beispielsweise um das Ein- und Ausführen der Sonde in das Bohrloch zu erleichtern. Der Durchmesser des Sondenkörpers 110 kann unterhalb von 76 mm liegen, um vorteilhafterweise ein Einführen der Sonde in ein Bohrloch mit einem Durchmesser von 76 mm (NQ) zu ermöglichen. Beispielsweise kann der Durchmesser der Sonde 50 mm bis 60 mm betragen, wie beispielsweise etwa 54 mm. Die Länge der Sonde 100 kann beispielsweise 2 m bis 3 m betragen, beispielsweise 2,5 m bis 2,75 m, beispielsweise etwa 2,66m. In Ausführungsformen kann der Sondenkörper 110 die Endplatten 130, 132 umfassen.

Der Sondenkörper 110 umfasst zumindest eine Öffnung 112. Wie in Fig. 1 und Fig. 2 gezeigt, kann der Sondenkörper 110 eine Vielzahl von Öffnungen 112 umfassen. Die Öffnungen 112 können sich über einen wesentlichen Abschnitt der Außenfläche des Sondenkörpers 110 erstrecken, beispielsweise einen wesentlichen Abschnitt der Mantelfläche der im Wesentlichen zylindrischen Sonde 100. Die Öffnungen 112 können beispielsweise als Bohrungen oder Schlitze gestaltet sein. Die Öffnungen 112 sind so gestaltet, dass ein Fluid 210, beispielsweise eine in einem Bohrloch vorhandene Flüssigkeit wie etwa Grundwasser, von einem Außenraum des Sondenkörpers in den Innenraum 114 des Sondenkörpers eingelassen wird. Vorteilhafterweise kann die Flüssigkeit durch die Öffnungen 112 in den Innenraum 114 einströmen, insbesondere sodass die Flüssigkeit im Innenraum 114 im Wesentlichen der Flüssigkeit im Außenraum des Sondenkörpers 110 entspricht.

Die Sonde 100 umfasst einen in dem Innenraum 114 liegenden Gaspermeator 120. Der Gaspermeator 120 umfasst in der gezeigten Ausführungsform mehrere Sintermetallträger 122 mit einem innenliegenden Gaskanal 126, insbesondere einem innerhalb des Sintermetallträgers 122 liegenden Gaskanal 126, sowie eine außenseitig auf dem Sintermetallträger 122 angeordnete, semipermeable Membran 124. Der Gaspermeator 120 ist für eine Diffusion von Gasen von dem Fluid 210 in den innenliegenden Gaskanal 126 eingerichtet.

Die Sonde 100 kann in ein Bohrloch 200 eingeführt werden. An einer dafür vorgesehenen Stelle innerhalb des Bohrlochs 200 kann ein sich in dem Bohrloch 200 befindendes Fluid 210, beispielsweise Grundwasser, durch die zumindest eine Öffnung 112 in den Innenraum 114 einströmen. Das Fluid 210 gelangt im Innenraum 114 in Kontakt mit dem Gaspermeator 120. Die in dem Fluid gelösten Gase können über die semipermeable Membran 124 und den Sintermetallträger 122 in den Gaskanal 126 diffundieren und somit zumindest anteilig ein Teil des in dem Gaskanal 126 befindlichen Gasgemisches ausbilden. Vorteilhafterweise kann das Gasgemisch analysierbar sein, beispielsweise um Rückschlüsse auf die Konzentration und/oder Zusammensetzung der in dem Fluid 210 gelösten Gase ziehen zu können.

In der gezeigten Ausführungsform ist der Sintermetallträger 122 ein rohrförmiger Sintermetallfilter mit einem innenliegenden Gaskanal 126. In der gezeigten Ausführungsform ist sie semipermeable Membran 124 ein Silikonschlauch, der über die Außenfläche des Sintermetallfilters 122 gestülpt ist.

Wie in Fig. 1 und 2 gezeigt umfasst der Gaspermeator 120 in der gezeigten Ausführungsform acht zylindrisch geformte Sintermetallträger 122 und jeweils eine die Außenfläche des jeweiligen Sintermetallträgers 122 umspannende semipermeable Membran 124. Die jeweiligen Sintermetallträger 122 sind als Hohlzylinder gestaltet, wobei der Hohlraum den Gaskanal 126 zumindest abschnittsweise ausbildet. In der gezeigten Ausführungsform umfasst der Gaspermeator 120 acht parallel verlaufende Sintermetallträger. Der Gaskanal 126 verläuft jeweils zwischen den offenen Enden 128 des Sintermetallträgers 126.

In weiteren Ausführungsformen kann die Form und Anzahl von Komponenten des Gaspermeators 120 abgeändert sein. Der Sintermetallträger 122 kann als Hohlkörper gestaltet sein. Die semipermeable Membran kann eine Außenfläche des Sintermetallträgers umspannen. Beispielsweise kann der zumindest eine Sintermetallträger als ein Prisma, beispielsweise mit einer sechs- oder acht-eckigen Grundfläche, oder einer Grundfläche in Form eines n-Ecks mit n ≥ 4 gestaltet sein.

In Ausführungsformen kann der Sintermetallträger 122, insbesondere ein rohrförmig gestalteter Sintermetallträger und/oder ein prismatischer Sintermetallträger, eine Wanddicke und einen Außendurchmesser aufweisen. Die Wanddicke kann 1 mm bis 5 mm betragen, beispielsweise 1,5 mm bis 3 mm, beispielsweise etwa 2 mm. Der Außendurchmesser kann 5 mm bis 20 mm betragen, beispielsweise 7,5 mm bis 15 mm, beispielsweise etwa 10 mm. Beispielsweise kann ein Sintermetallträger 122 eine Wanddicke von 2mm und einen Außendurchmesser von 10 mm haben.

Insbesondere können mehr oder weniger als acht Sintermetallträger vorgesehen sein. Die Anzahl von Sintermetallträgern 122 kann geradzahlig sein. Durch die Verwendung von einer nebeneinander angeordneten, geradzahligen Anzahl von Sintermetallträgern 122 kann erreicht werden, dass der Gaskanal 126 des Gaspermeators in derselben Endplatte 130 sowohl einen Einlass als auch einen Auslass aufweist. Beispielsweise kann der Gaspermeator 120 zwischen zwei und acht Sintermetallträger 122 umfassen. Beispielsweise kann für Sonden, die in Bohrlöcher mit einem größeren Durchmesser als 76 mm eingesetzt werden sollen, die Anzahl von Sintermetallträgern 122 auf mehr als acht, beispielsweise gleich oder mehr als 10, gleich oder mehr als 12, oder sogar gleich oder mehr als 14 erhöht werden. Beispielsweise kann eine Anzahl von Sintermetallträgern 122 auf weniger als acht, beispielsweise gleich oder weniger als 6, gleich oder weniger als 4, oder sogar 2 verringert werden, beispielsweise um das Volumen und/oder den Strömungsquerschnitt des Gaskanals 126 durch Vergrößerung des Durchmessers des Sintermetallträgers zu erhöhen.

Wie in Fig. 1 und Fig. 2 gezeigt umfasst die Sonde zwei Endplatten 130 und 132. Verbindungsbohrungen 220 innerhalb der Endplatten 130, 132 sind dazu vorgesehen, den Gaskanal 126 der jeweiligen Sintermetallträger 122 miteinander zu verbinden, und somit einen gemeinsamen Gaskanal 126 des Gaspermeators 120 auszubilden. Zusätzlich sind in der oberen Endplatte 130 Verbindungsbohrungen 220 vorgesehen, um den Gaskanal 126 mit einem in der Endplatte 130 vorgesehenen Einlassstück und Auslassstück zu verbinden. Wie in Fig. 1 und 2 gezeigt kann der Gaskanal 126 durch eine serielle Verbindung der jeweiligen Sintermetallträger 122 mit den Verbindungsbohrungen 220 ausgebildet sein. In weiteren Ausführungsformen kann auch ein verzweigter Gaskanal 126 ausgebildet werden, beispielsweise durch paralleles Verbinden von zwei oder mehr Sintermetallträgern 122.

In Ausführungsformen ist die Sonde 100 dazu eingerichtet, fluidisch mit einem Gasanalysator verbunden zu werden. Insbesondere kann der Gaskanal 126 des Gaspermeators fluidisch mit einem Gasanalysator verbindbar sein. Beispielsweise können in einer Endplatte 130 der Sonde Ein- und Auslasstücke vorgesehen sein, die an ein Gaszuleitungen und/oder Gasableitungen eines Gasanalysators anschließbar sind.

In Ausführungsformen ist der Gaskanal 126 von einem Trägergas durchströmbar. Das Trägergas kann beispielsweise ein von einem Gasanalysesystem bereitgestelltes Trägergas sein. Beispielsweise kann das Trägergas in der Sonde 100 mit Bestandteilen der in dem Fluid gelösten Gase als Analyten durchmischt werden. Die Analyten können durch Förderung des Trägergases aus der Sonde 100 in das Gasanalysesystem befördert und dort analysiert werden.

Vorteilhafterweise kann die Sonde Gase eines mit Gas gesättigten oder übersättigten Fluids ebenso wie ein mit Gas untersättigtes Fluid analysieren. Insbesondere kann die Sonde dazu geeignet sein, eine Analyse mit vergleichsweise niedrigen Gesamtgasdrücken durchzuführen, beispielsweise mit Gesamtgasdrücken von weniger als 1 bar.

In Ausführungsformen kann die Sonde 100 einen Temperatursensor 140 umfassen. Der Temperatursensor 140 kann beispielsweise in dem Innenraum 114 angeordnet sein. Der Temperatursensor 140 kann gleichermaßen im Bereich einer der Endplatten 130, 132 angeordnet sein. Der Temperatursensor 140 kann über eine Stromversorgungs- und/oder Signalleitung (nicht gezeigt) verbunden oder verbindbar sein. Die Stromversorgungs- und/oder Signalleitung kann mit einem Gasanalysesystem verbunden sein, beispielsweise um eine Temperatur der Sonde 100 und/oder des Fluids 210 in ein Analyseergebnis einbeziehen zu können.

In Ausführungsformen kann die Sonde 100 einen Drucksensor 150 umfassen. Der Drucksensor kann fluidisch mit dem Gaskanal 126 und/oder dem Fluid 210 verbunden sein. Beispielsweise kann der Drucksensor 150 im Bereich einer der Endplatten 130, 132 angeordnet sein. Beispielsweise kann der Drucksensor über eine Verbindungsbohrung ähnlich der in Fig. 2 gezeigten Verbindungsbohrung 220 mit dem Gaskanal verbunden sein. Der Drucksensor kann über eine Stromversorgungs- und/oder Signalleitung (nicht gezeigt) verbunden oder verbindbar sein. Die Stromversorgungs- und/oder Signalleitung kann mit einem Gasanalysesystem verbunden sein, beispielsweise um einen Druck des Gasgemisches in dem Gaskanal 126 in ein Analyseergebnis einbeziehen zu können. Alternativ oder zusätzlich kann der Druckmesser mit dem Innenraum 114 verbunden sein, beispielsweise um einen Druck des Fluids 210 zu bestimmen und den Druck des Fluids in ein Analyseergebnis einbeziehen zu können.

In Ausführungsformen kann die Sonde 100 ein Probenahmesystem 160 umfassen. Das Probenahmesystem 160 kann dazu eingerichtet sein, eine Probe des im Innenraum 114 vorhandenen Fluids 210 zu nehmen und einen Transport des Fluids zusammen mit der Sonde 100 an die Oberfläche zu ermöglichen. Dies kann vorteilhafterweise eine Analyse des Fluids 210 mit konventionellen Methoden anstelle oder zusätzlich zur *in situ* Analyse ermöglichen. Das Probenahmesystem 160 kann einen Behälter umfassen. Der Behälter kann innerhalb des Innenraums 114 vorgesehen sein und für ein Einströmen des Fluids 210 eingerichtet sein. Der Behälter kann verschließbar sein. Das Probenahmesystem kann über eine Stromversorgungs- und/oder Signalleitung (nicht gezeigt) verbunden oder verbindbar sein, beispielsweise um eine Öffnung oder ein Ventil des Behälters des Probenahmesystems 160 zu steuern.

In Ausführungsformen kann die Sonde 100 ein Probenahmesystem 160 umfassen. Das Probenahmesystem 160 kann dazu eingerichtet sein, eine Probe des im Gaskanal vorhandenen Gasgemisches zu nehmen und einen Transport des Gasgemisches zusammen mit der Sonde 100 an die Oberfläche zu ermöglichen. Dies kann vorteilhafterweise eine Analyse des Gasgemisches mit konventionellen Methoden anstelle oder zusätzlich zur *in situ* Analyse ermöglichen. Das Probenahmesystem 160 kann einen Behälter umfassen. Der Behälter kann innerhalb des Innenraums 114 vorgesehen sein und für ein Einströmen des Gasgemisches eingerichtet sein. Der Behälter kann verschließbar sein. Das Probenahmesystem kann über eine Stromversorgungs- und/oder Signalleitung (nicht gezeigt) verbunden oder verbindbar sein, beispielsweise um eine Öffnung oder ein Ventil des Behälters des Probenahmesystems 160 zu steuern.

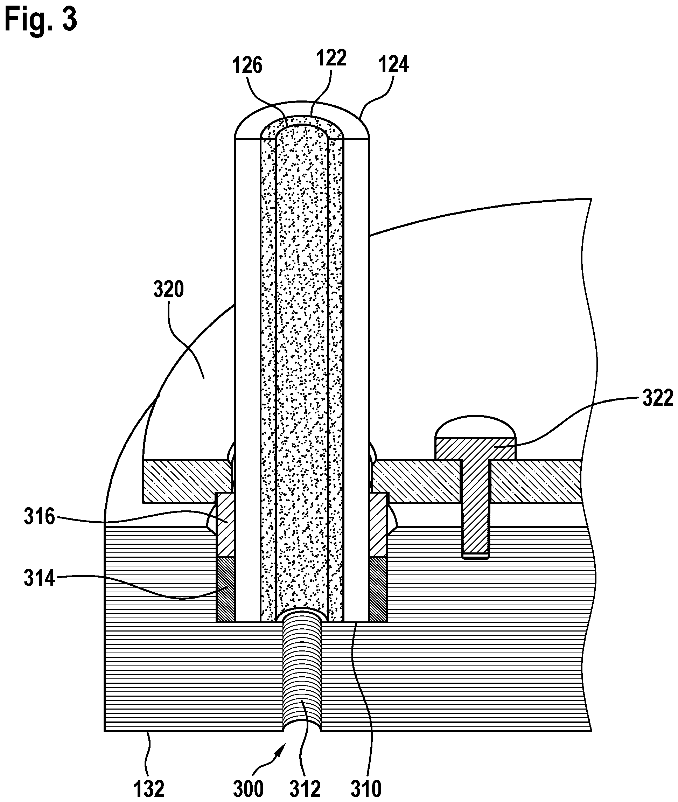

In Fig. 3 ist eine Ausführungsform einer Endplatte 300 im Detail beschrieben. Die in Fig. 3 gezeigte Endplatte 300 kann beispielsweise eine der in Fig. 1 gezeigte Endplatte 132 oder eine Endplatte 130 (nicht gezeigt) umfassen. In Fig. 3 ist des Weiteren ein Gaspermeator gezeigt. Der Gaspermeator kann ein Gaspermeator 120, oder ein Teil eines Gaspermeators 120, gemäß der in Fig. 1 beschriebenen Ausführungsform sein. Der Gaspermeator umfasst einen Sintermetallträger 122 mit einem Gaskanal 126 und eine außenseitig angebrachte, semipermeable Membran 124.

In der Endplatte 132 ist eine Öffnung 310 vorgesehen. Der Gaspermeator ist teilweise in die Öffnung 310 eingeführt. In der Öffnung 310 sind eine Dichtung 314 und eine Hülse 316 vorgesehen. Die Dichtung 314 und die Hülse 316 sind über einen Abschnitt des Gaspermeators gestülpt, sodass die Dichtung 314 und die Hülse in radialer Richtung der Öffnung 310 und/oder des Gaspermeators zwischen der semipermeable Membran 124 und der Öffnung 310 liegen.

Eine Endplatte 320 ist mit zumindest einem Befestigungselement 322, beispielsweise einer Schraube, an der dem Innenraum der Sonde zugewandten Seite der Endplatte befestigt. Durch die Befestigung mit dem Befestigungselement 322 wird die Gegenplatte 322 an die Endplatte gepresst 132 und die Hülse 316 in die Öffnung gepresst. Die Hülse 316 presst gegen die Dichtung 314 und quetscht die Dichtung 314, sodass sich die Dichtung 314 in radiale Richtung ausdehnt. Durch die Verformung der Dichtung wird die semipermeable Membran 124 gegenüber der Mantelfläche der Öffnung 310 abgedichtet. Somit kann ein Eintritt des Fluids aus dem Innenraum der Sonde in den Gaspermeator verhindert werden.

Wie in Fig. 3 gezeigt umfasst die Endplatte 132 einen Kanal 312. Der Kanal 312 kann beispielsweise ein Abschnitt einer der in Fig. 2 gezeigten Verbindungsbohrungen 220 sein, und/oder zu einer Anschluss- oder Verbindungsbohrung 220 führen.

In Ausführungsformen kann die in Fig. 3 gezeigte Endplatte 300 in gleicher oder ähnlicher Weise an beiden Enden einer Sonde, wie etwa der Sonde 100 vorgesehen sein, sodass beispielsweise ein Gaspermeator beidseitig in den jeweiligen Endplatten 300 fixiert und/oder abgedichtet ist und insbesondere ein Gaskanal 126 des Gaspermeators in der Endplatte über den Kanal 312 weitergeführt werden kann.

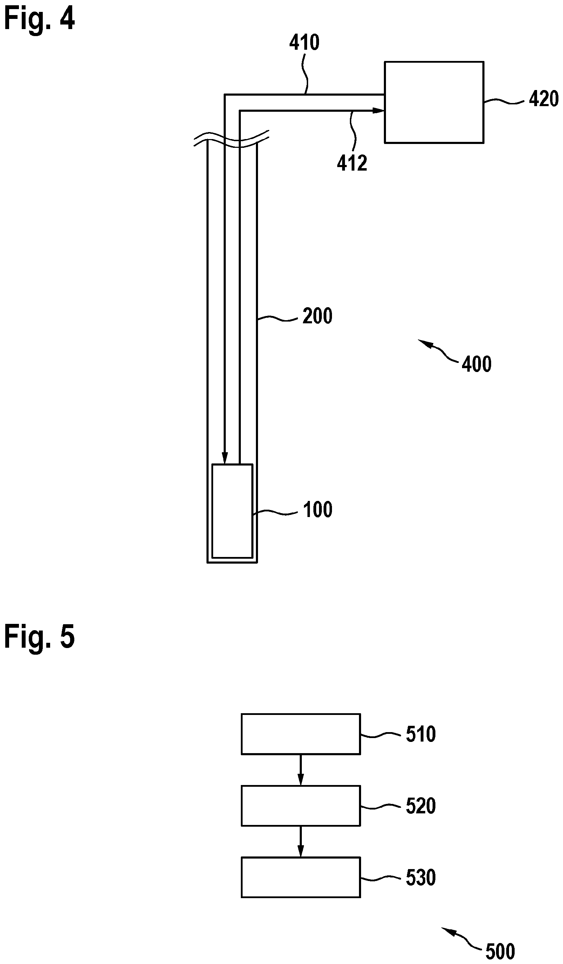

In Fig. 4 ist ein Gasanalysesystem 400 gezeigt. Das Gasanalysesystem 400 umfasst eine Sonde 100 gemäß hierin beschriebenen Ausführungsformen. Die Sonde 100 ist in ein Bohrloch 200 eingeführt, beispielsweise ein Bohrloch 200 mit einer Tiefe von mehr als 500 m. In dem Bohrloch befindet sich ein Fluid, wie etwa das Fluid 210, wie etwa Grundwasser. In dem Fluid gelöste Gase diffundieren über den Gaspermeator der Sonde 100 in den Gaskanal 126 des Gaspermeators. Der Gaskanal 126 ist fluidisch mit einer Gaszuleitung 410 und einer Gasableitung 412 verbunden, sodass ein Einpumpen von Gas über die Gaszuleitung 410 zu einem Ausströmen von Gas über die Gasableitung 412 führt. Das über die Gasableitung 412 rückgeführte Gas ist durch den Gasanalysator 420 analysierbar. Bei dem in die Gaszuleitung 410 eingepumpten Gas kann es sich um ein Trägergas handeln.

Wie in Fig. 4 gezeigt ist der Gasanalysator 420 außerhalb des Sondenkörpers 110 der Sonde 100 angeordnet. Der Gasanalysator 420 kann zudem außerhalb des Bohrlochs 200 positioniert sein. Der Gasanalysator 420 kann dazu eingerichtet sein, das Trägergas bereitzustellen. Beispielsweise kann der Gasanalysator 420 eine Pumpe beinhalten, die ein Trägergas mit einem Druck in die Gaszuleitung 410 pumpt und/oder das Trägergas zirkuliert. Der Druck kann beispielsweise eine Differenzdruck von etwa 1 bar, 2 bar, 3 bar, 4 bar oder sogar 5 bar zwischen der Gaszuleitung 410 und der Gasableitung 412 im Gasanalysator 420 sein.

In weiteren Ausführungsformen kann das Gas und/oder Trägergas von einem weiteren System, beispielsweise einem externen Pumpsystem, bereitgestellt werden, sodass der Gasanalysator nur mit der Gasableitung 412 fluidisch verbunden ist.

In Ausführungsformen kann das Trägergas, beispielsweise Argon, beispielsweise aus einer Druckgasflasche stammen und mit einem definierten Druck, beispielsweise einem durch ein Druckreduzierventil regulierten Druck, in die Gaszuleitung 410 eingespeist werden.

In weiteren Ausführungsformen kann auf die Gaszuleitung 410 verzichtet werden. Beispielsweise kann die Ableitung 412 vor dem Einsatz der Sonde mit einem Trägergas gefüllt werden. Durch die Diffusion von Gasen in den Gaskanal 126 können die Gase einen Druck innerhalb des Gaskanals 126 aufbauen, sodass die Gase passiv über die Ableitung 412 ausströmen.

In Ausführungsformen kann der Gasanalysator 420 mit einem oder mehreren der in Zusammenhang mit Fig. 1 beschriebenen Signalleitungen, beispielsweise des Temperatursensors 140, des Drucksensors 150 und/oder des Probenahmesystems 160 verbunden sein.

Der Gasanalysator 420 ist dazu eingerichtet, das aus der Sonde 100 rückgeführte Gas, insbesondere ein Trägergas, zu analysieren, das mit Analyten, insbesondere den aus dem Fluid in den Gaskanal des Gaspermeators diffundierten Gasen, angereichert ist. Eine Analyse kann insbesondere eine quantitative Bestimmung der Analyten beinhalten. Bei dem Gasanalysator 420 kann es sich um einen bekannten Gasanalysator handeln, beispielsweise um einen Analysator auf Basis von Massenspektrometrie, Infrarotspektrometrie wie beispielsweise Fourier-transformierte Infrarotspektrometrie, Raman-Spektrometrie, Gaschromatographie oder weiteren bekannten Analysemethoden.

In Ausführungsformen kann der Gasanalysator 420 mehrere Analysemethoden kombinieren und/oder aus mehreren Gasanalysator-Modulen bestehen. Beispielsweise kann für jedes zu analysierende Gas ein dediziertes Gasanalysator-Modul bereitgestellt werden Die mehreren Gasanalysator-Module können beispielsweise in Serie geschaltet sein, und/oder durch Ventile einkoppelbar sein.

In Ausführungsformen kann der Gasanalysator 420 eine Wasserfalle beinhalten, beispielsweise um Wasserdampf aus dem in der Gasableitung 412 rückgeführten Gas vor der Analyse zu entfernen.

In Ausführungsformen kann der Gasanalysator 420 einen Drucksensor beinhalten, beispielsweise um eine Druckdifferenz zwischen der Gaszuleitung 410 und der Gasableitung 412 zu bestimmen.

In Ausführungsformen kann der Gasanalysator 420 einen Durchflusssensor beinhalten, beispielsweise um die Flussrate des Gases in der Gaszuleitung 410 und/oder der der Gasableitung 412 zu bestimmen.

Vorteilhafterweise ist das Gasanalysesystem 400 dazu geeignet, die Zusammensetzung des Fluids 210 über einen längeren Zeitraum oder sogar dauerhaft zu überwachen. Die Sonde 100 kann dazu vorteilhafterweise in dem Bohrloch 200 verbleiben, sodass der Aufwand einer dauerhaften Messung gegenüber einem System, das eine wiederholte Probenahme vorsieht, verringert wird.

In Ausführungsformen kann das Gasanalysesystem 400 vorteilhafterweise die sofortige und/oder die wiederholte Messung über variable Zeiträume ermöglichen. Beispielsweise kann eine Messung in beliebigen Zeitintervallen erfolgen, beispielsweise in Intervallen zwischen einer Minute und über einen Zeitraum von einem Jahr oder länger. Vorteilhafterweise können die erfassten Daten Informationen über die Temperatur im Bohrloch, wahlweise in Relation zu einer Temperatur an der Oberfläche, umfassen. Vorteilhafterweise können die erfassten Daten Druckdaten innerhalb des Bohrlochs (Gesamtdruck), sowie in Ausführungsformen Druckdaten des in einem Oberflächenmodul gemessenen Drucks umfassen. Vorteilhafterweise können die erfassten Daten Informationen über die Art der nachgewiesenen Gase, der Anteile der jeweils nachgewiesenen Gase, beispielsweise ausgedrückt als Konzentrationen oder Partialdrücke, umfassen.

In Fig. 5 ist ein Verfahren 500 zur Gasanalyse in einem Bohrloch gezeigt. Das Bohrloch kann ein Bohrloch gemäß der hierin beschriebenen Ausführungsformen und/oder Aspekte sein.

Das Verfahren 500 umfasst das Einführen 510 einer Sonde 100 gemäß einer der hierin beschriebenen Ausführungsform in das Bohrloch an eine vorgegebene Position. Die vorgegebene Position kann beispielsweise eine Position in einer Tiefe von mehr als 500 m, mehr als 600 m, mehr als 700 m, mehr als 800 m, mehr als 900 m, oder sogar mehr als 1000 m sein. Die Sonde 100 kann beispielsweise mit einem Kabel und einer Winde in vertikalen Bohrlöchern installiert werden. Die Sonde 100 kann beispielsweise mit Installationsstangen in subhorizontalen, horizontalen oder nach oben geneigten Bohrlöchern installiert werden.

Das Verfahren 500 umfasst das Abwarten 520 einer Diffusion von Gasen von einem in dem Bohrloch 200 an der vorgegebenen Position vorhandenen Fluid 210 über die semipermeable Membran 124 in den Gaskanal 126 der Sonde 100. Das Abwarten kann beispielsweise eine Zeitspanne umfassen, in der sich ein Konzentrationsgleichgewicht und/oder ein Partialdruckgleichgewicht zwischen dem Fluid 210 und dem Gaskanal 126 einstellt.

In Ausführungsformen kann während des Abwartens 520 ein Fluss eines Trägergases gestoppt sein, sodass sich Analyten in dem Gaskanal 126 anreichern können, beispielsweise bis zum Erreichen eines zumindest teilweisen Partialdruckgleichgewichts.

In weiteren Ausführungsformen kann während des Abwartens 520 ein Trägergas mit einer vordefinierten Flussrate durch den Gaskanal 126 strömen. Beispielsweise kann das Abwarten 520 eine Beobachtung der in einem Analysesystem bestimmten Messwerte und/oder eine Stabilisation der in einem Analysesystem bestimmten Messwerte umfassen. Beispielsweise kann das Abwarten eine Stabilisierung des mit dem Drucksensor 150 gemessenen Drucks beinhalten.

In weiteren Ausführungsformen kann während des Abwartens 520 das Trägergas durch die Sonde 100 und den Gasanalysator 420 zirkuliert werden, bis sich ein Gleichgewicht eingestellt hat.

In weiteren Ausführungsformen kann auf die Zirkulation des Trägergases zumindest zeitweise verzichtet werden. Das Abwarten 520 kann beispielsweise die Beobachtung des mit dem Drucksensor 150 gemessenen Drucks innerhalb des Gaskanals umfassen, insbesondere eine Stabilisierung des Drucks. Sobald sich der Druck stabilisiert hat, kann davon ausgegangen werden, dass sich innerhalb des Gaskanals ein Gleichgewicht eingestellt hat. Sobald sich das Gleichgewicht eingestellt hat, kann eine Analyse 530 durchgeführt werden.

Das Verfahren 500 umfasst die Analyse 530 des in dem Gaskanal 126 vorhandenen Gasgemisches. Die Analyse kann beispielsweise in einem Gasanalysesystem 400 mit einem Gasanalysator 420 gemäß hierin beschriebenen Ausführungsformen durchgeführt werden.

In Ausführungsformen kann die Analyse 530 das Einleiten eines Trägergases in einen fluidisch mit dem Gaskanal 126 verbundenen Kanal, beispielsweise eine Gaszuleitung 410, umfassen. Die Analyse 530 kann den Transport des Gasgemisches zu einem außerhalb des Bohrloches 200 angeordneten Gasanalysators 420 in Folge des Einleitens des Trägergases, beispielsweise in der Gasableitung 412, umfassen.

In Ausführungsformen kann das in den Gaskanal 126 diffundierte Gas, beispielsweise nach dem Erreichen eines Druckgleichgewichts, über das Probenahmesystem 160 als Probe genommen werden und anschließend, beispielsweise nach dem Transport der Sonde 100 an die Oberfläche, analysiert werden.

Die hierin beschriebenen Ausführungsformen erlauben eine *in situ* Gasanalyse in tiefen Bohrlochern selbst bei hohen Temperaturen und Drücken. Vorteilhafterweise kann die Sonde über einen längeren Zeitraum in dem Bohrloch verbleiben und vorzugsweise für eine dauerhafte, konstante Beobachtung der Bedingungen im Bohrloch eingesetzt werden. Das Gasanalysesystem kann modular aufgebaut sein und für die jeweils zu bestimmenden Gase angepasst und beliebig erweitert werden. Durch die Positionierung des Gasanalysators außerhalb des Bohrlochs ist der Gasanalysator nicht in Größe und Beschaffenheit beschränkt. Ausführungsformen des hierin beschriebenen Gaspermeators haben den Vorteil, bei geringer Abmessung, insbesondere einem geringen Durchmesser der Sonde, eine hohe Oberfläche bereitzustellen und somit einen schnellen Gasaustausch zu ermöglichen. Die hierin beschriebene Sonde ist vorteilhafterweise für die Verwendung in Bohrlöchern mit geringem Durchmesser geeignet, sodass Bohrungen mit geringerem Aufwand durchgeführt werden können.

Die hierin beschriebenen, spezifischen Ausführungsformen sind Beispiele und sollen nicht als einschränkend verstanden werden. Dem Fachmann ist es ersichtlich, dass zahlreiche Modifikationen der Form, Struktur und Zusammensetzung möglich sind, ohne den Rahmen der Erfindung zu verlassen.

## Patentansprüche

1. Sonde (100) zur Gasanalyse in Bohrlöchern (200), umfassend:
einen in das Bohrloch (200) einführbaren Sondenkörper (110) mit zumindest einer Öffnung (112) zum Einlassen von Fluid (210) aus einem Außenraum des Sondenkörpers (110) in einen Innenraum (114) des Sondenkörpers (110);
einen in dem Innenraum (114) des Sondenkörpers (110) vorgesehenen Gaspermeator (120), umfassend einen Sintermetallträger (122) mit einem innenliegenden Gaskanal (126) und eine außenseitig auf dem Sintermetallträger (122) angeordnete, semipermeable Membran (124),
wobei der Gaspermeator (120) zur Diffusion von Gasen von dem Fluid (210) in den innenliegenden Gaskanal (126) eingerichtet ist.

2. Sonde (100) nach Anspruch 1, wobei der Sintermetallträger (122) als Hohlkörper gestaltet ist, welcher eine von der semipermeablen Membran (124) umspannte Außenfläche aufweist.

3. Sonde (100) nach Anspruch 1 oder 2, wobei der Gaskanal (126) sich entlang einer Achse zwischen einem offenen ersten Ende (128) und einem offenen zweiten Ende (128) des Sintermetallträgers (122) erstreckt.

4. Sonde (100) nach einem der vorangehenden Ansprüche, wobei der Gaspermeator (120) zumindest zwei Sintermetallträger (122) umfasst.

5. Sonde (100) nach einem der vorangehenden Ansprüche, wobei der Gaskanal (126) dazu eingerichtet ist, fluidisch mit einem Gasanalysator (420) verbunden zu sein.

6. Sonde (100) nach einem der vorangehenden Ansprüche, wobei der Gaskanal (126) von einem Trägergas durchströmbar ist.

7. Sonde (100) nach einem der vorangehenden Ansprüche mit einer Endplatte (130, 132), wobei der Gaspermeator (120) an zumindest einem ersten Ende an der Endplatte (130, 132) befestigt ist, die Endplatte (130, 132) umfassend:
eine Öffnung (310) zur teilweisen Aufnahme des Gaspermeators (120);
einen fluidisch mit dem Gaskanal (126) verbundenen Kanal (312);
eine Dichtung (314), wobei die Dichtung (314) zwischen der Öffnung (310) und der semipermeablen Membran (124) angeordnet ist.

8. Sonde (100) nach einem der vorangehenden Ansprüche, wobei der Sondenkörper (110) einen Außendurchmesser von maximal 76 mm aufweist.

9. Sonde (100) nach einem der vorangehenden Ansprüche, wobei der Sintermetallträger (122) aus einem gesinterten Edelstahl gefertigt ist.

10. Sonde (100) nach einem der vorangehenden Ansprüche, wobei die semipermeable Membran (124) aus einem Silikonschlauch gefertigt ist.

11. Sonde (100) nach einem der vorangehenden Ansprüche, wobei innerhalb des Sondenkörpers (110) zumindest eines angeordnet ist von:
ein Temperatursensor (140);
ein mit dem Gaskanal (126) und/oder dem Fluid (210) fluidisch verbundener Drucksensor (150); und
ein mit dem Gaskanal (126) und/oder dem Fluid (210) fluidisch verbundenes Probenahmesystem (160).

12. Gasanalysesystem (400), umfassend eine Sonde (100) nach einem der vorhergehenden Ansprüche mit einem Gasanalysator (420), welcher außerhalb des Sondenkörpers (110) angeordnet ist.

13. Verfahren (500) zur Gasanalyse in einem Bohrloch (200) mit einer Sonde (100) nach einem der Ansprüche 1 bis 11, umfassend:
Einführen (510) der Sonde in das Bohrloch (200) an eine vorgegebene Position;
Abwarten (520) einer Diffusion von Gasen von einem in dem Bohrloch (200) an der vorgegebenen Position vorhandenen Fluid (210) über die semipermeable Membran (124) in den Gaskanal (126) der Sonde; und
Analyse (530) des in dem Gaskanal (126) vorhandenen Gasgemisches.

14. Verfahren (500) nach Anspruch 13, wobei die Analyse (530) des in dem Gaskanal (126) vorhandenen Gasgemisches umfasst:
Einleiten eines Trägergases in einen fluidisch mit dem Gaskanal (126) verbundenen Kanal (410, 412); und
Transport des Gasgemisches zu einem außerhalb des Bohrloches (200) angeordneten Gasanalysators (420).

15. Verfahren nach einem der Ansprüche 13 bis 14, wobei die vorgegebene Position in einer Tiefe von mehr als 500m liegt und/oder an der vorgegebenen Position eine Temperatur von mindestens 50°C herrscht und/oder ein Druck von mindestens 50 bar herrscht.
